(19) Europäisches Patentamt
European Patent Office
Office européen des brevets

(11) Publication number: **0 308 961 B1**

(12) **EUROPEAN PATENT SPECIFICATION**

(45) Date of publication of patent specification: **08.07.92**

(51) Int. Cl.[5]: **C07D 285/34**, A01N 43/88

(21) Application number: **88115697.0**

(22) Date of filing: **23.09.88**

(54) **Thiadiazines, process for production thereof, and insecticide.**

(30) Priority: **25.09.87 JP 239002/87**
**14.07.88 JP 176063/88**

(43) Date of publication of application:
**29.03.89 Bulletin 89/13**

(45) Publication of the grant of the patent:
**08.07.92 Bulletin 92/28**

(84) Designated Contracting States:
**CH DE ES FR GB IT LI NL**

(56) References cited:

**CHEMICAL ABSTRACTS, vol. 106, no. 7, February 16, 1987, Columbus, Ohio, USA; ISHII S. et al.: "1,3,5-thiadiazin-4-ones as acaricides and insecticides", page 650, column 1, abstract-no. 50 258t**

**CHEMICAL ABSTRACTS, vol. 97, no. 1, July 5, 1982, Columbus, Ohio, USA; NIHON NOYAKU CO. LTD.: "Tetrahydrothiadiazones as acaricides", page 239, column 1, abstract-no. 2 276w**

(73) Proprietor: **MITSUI TOATSU CHEMICALS, Inc.**
**2-5 Kasumigaseki 3-chome**
**Chiyoda-Ku Tokyo 100(JP)**

(72) Inventor: **Nakaya, Michihiko**
**4-10-8, Hisagi,**
**Zushi-shi Kanagawa-ken(JP)**
Inventor: **Fukushi, Yukiharu**
**3-42-7-128, Hirado, Totsuka-ku**
**Yokohama-shi Kanagawa-ken(JP)**
Inventor: **Shiraishi, Shirou**
**Mitsui Toatsu Shataku 2-1-3, 1541 Yabe-cho,**
**Totsuka-ku, Yokohama-shi**
**Kanagawa-ken(JP)**
Inventor: **Nakamura, Masahiko**
**Mitsui Toatsu Iijima Shataku 4-37, 2882**
**Iijima-cho**
**Sakae-ku, Yokohama-shi Kanagawa-ken(JP)**
Inventor: **Numata, Satoshi**
**Mitsui Toatsu Shataku 1-4-1, 1541 Yabe-cho,**
**Totsuta-ku, Yokohama-shi Kanagawa-ken(JP)**
Inventor: **Kodaka, Kenji**
**Mitsui Toatsu Iijima Shataku 1-22, 2882**
**Iijima-cho**
**Sakae-ku, Yokohama-shi Kanagawa-ken(JP)**

EP 0 308 961 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

**CHEMICAL ABSTRACTS, vol. 93, no. 5, August 4, 1980, Columbus, Ohio, USA; IKEDA K. et al.: "Thiadiazines", page 941, column 1, abstract-no. 46 729t**

**CHEMICAL ABSTRACTS, vol. 91, no. 11, September 10, 1979, Colombus, Ohio, USA; IKEDA K. et al.: "Thiadiazines", page 785, column 2, abstract-no. 91 678v**

Inventor: **Ooka, Masayuki**
**Mitsui Toatsu Iijima Shataku 2-25, 2882**
**Iijima-cho**
**Sakae-ku, Yokohama-shi Kanagawa-ken(JP)**

(74) Representative: **Zumstein, Fritz, Dr. et al**
**Dr. F. Zumstein Dipl.-Ing. F. Klingseisen**
**Bräuhausstrasse 4**
**W-8000 München 2(DE)**

## Description

This invention relates to novel tetrahydro-1,3,5-thiadiazin-4-ones represented by general formula (I)

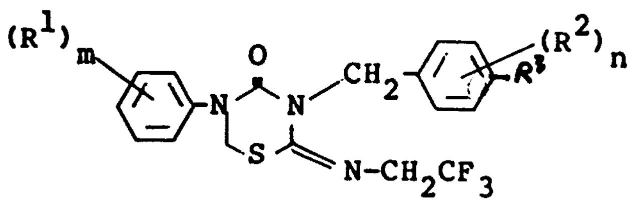

(I)

wherein each of $R^1$ and $R^2$ represents a halogen atom or an alkyl group having 1 to 4 carbon atoms, $R^3$ represents a halogen atom, or an alkyl group having 1 to 4 carbon atoms, a cycloalkyl group having 3 to 6 carbon atoms, an alkoxy group having 1 to 6 carbon atoms, an acetyl, a phenoxy, a halo-substituted phenoxy, a benzyl, a benzyloxy, a phenylcarbonyl or a trifluoromethyl group, m represents 0, 1, 2 or 3, and n represents 0, 1 or 2; and their salts; a process for production thereof; and an insecticide comprising at least one of these compounds as an active ingredient.

The compounds provided by this invention are useful in the agricultural field as insecticides.

Japanese Laid-Open Patent Publication No. 154780/1979 and US-A-4,159,328 describe that tetrahydro-1,3,5-thiadiazin-4-ones have insecticidal and miticidal activities. Among them, 2-tertiary butylimino-3-isopropyl-5-phenyl-tetrahydro-1,3,5-thiadiazin-4-one (common name: Buprofezin) of formula (IV) below has actually been used as an insecticide.

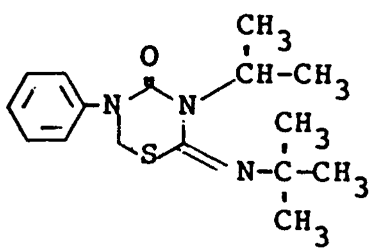

(IV)

Likewise from C.A. 91 (1979), 91678v; C.A. 97 (1982), 2276w and C.A. 93 (1980), 46729t further tetrahydro-1,3,5-thiadiazin-4-ones are known, useful as insecticides and miticides or acaricides respectively.

Japanese Laid-Open Patent Publication No. 140577/1986 (cf. C.A. 106 (1987), 50258t) states that tetrahydro-1,3,5-thiadiazin-4-ones in which at least one of the 2-imino group and the 3- and 5-positions is substituted by a certain substituted phenylalkyl group are novel compounds having insecticidal and acaricidal activities, and particularly those in which at least one of the 2-imino group and the 3-position is substituted by a certain substituted phenylalkyl group, showed marked insecticidal and acaricidal activities as compared with Buprofezin, a known compound. However, these insecticdal and acaricidal agents do not have sufficient insecticidal activity on lepidopterous insect pests although they have insecticidal activity on hemipterous and coleopterous insect pests. It was desired therefore, to develop a novel chemical which has similar activities to these insecticidal and acaricidal agents as well as having outstanding insecticidal activity on lepidopterous insect pests.

It is an object of this invention to provide an insecticidal compound having a novel structure, a broad insecticidal spectrum and high insecticidal activity, an insecticide comprising it, and a simple process for producing it, which give a solution to the aforesaid problem of the prior art.

The present inventors extensively studied tetrahydro-1,3,5-thiadiazin-4-ones in order to solve the above problem, and have now found that 2-(2,2,2-trifluoroethylimino)-tetrahydro-1,3,5-thiadiazin-4-ones and their salts have a broad insecticidal spectrum and a broad acaricidal spectrum, and high insecticidal activity on lepidopterous insect pests on which known analogous compounds do not show sufficient insecticidal activity.

Thus, the present invention provides a tetrahydro-1,3,5-thiadiazin-4-one represented by general formula (I)

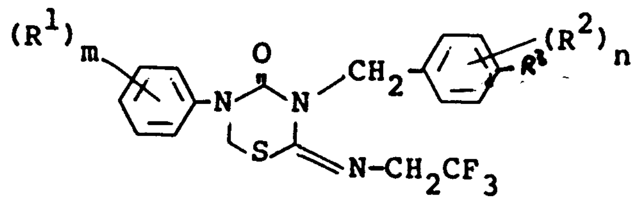 (I)

wherein each of $R^1$ and $R^2$ represents a halogen atom or an alkyl group having 1 to 4 carbon atoms, $R^3$ represents a halogen atom, or an alkyl group having 1 to 4 carbon atoms, a cycloalkyl group having 3 to 6 carbon atoms, an alkoxy group having 1 to 6 carbon atoms, an acetyl, a phenoxy, a halo-substituted phenoxy, a benzyl, a benzyloxy, a phenylcarbonyl or a trifluoromethyl group, m represents 0, 1, 2 or 3, and n represents 0, 1 or 2,

and its salts; a process for producing a tetrahydro-1,3,5-thiadiazin-4-one of general formula (I), which comprises reacting a compound of general formula (II)

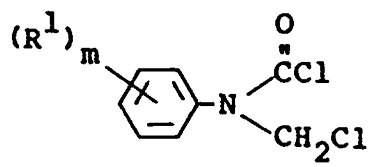 (II)

wherein $R^1$ and m are as defined above,

with a compound represented by general formula (III)

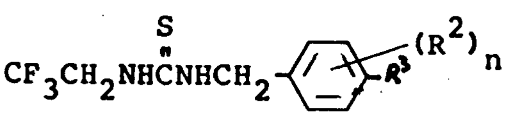 (III)

wherein $R^2$, $R^3$ and n are as defined above;

and an insecticide comprising an insecticidally effective amount of at least one tetrahydro-1,3,5-thiadiazin-4-one or its salts and a carrier.

The tetrahydro-1,3,5-thiadiazin-4-ones of general formula (I) and their salts are not described in the literature and are novel compounds.

If the 3-position benzyl group has two substituents, they are preferably at the 3,4-positions, and the substituent at the 3-position is preferably a halogen atom, especially a fluorine atom. When the 5-position phenyl group of the thiadiazine ring has one substituent, it is preferably a fluorine atom for the halogen, especially a fluorine atom at the 2-position, and a methyl group for the alkyl, especially a methyl group at the 3- or 4-position. If the 5-phenyl group has two substituents, they are preferably at the 2,4-positions, 3,4-positions or 2,6-positions. A methyl group is preferred as the 3-position substituent and the 4-position substituent, and a fluorine atom is preferred as the 6-position substituent. If the 5-phenyl group has three substituents, they are preferably at the 2,4,6-positions and 3,4,6-positions. In this case, a fluorine atom is preferred as the 2-position substituent, and a methyl group is preferred as the 3-position substituent and the 4-position substituent, and the 6-substituent is preferably a fluorine atom. When $R^3$ in the formula (I) represents a halo-substituted phenoxy group, the halogen substituent may be chlorine, bromine or fluorine.

Typical examples of the compound of general formula (I) are shown in Table 1.

Table 1

| Compound No. | $(R^1)_m$ | $(R^2)_n$ | $R^3$ |
|---|---|---|---|
| 1 | H | H | $CH_3$ |
| 2 | H | H | $C_2H_5$ |
| 3 | H | H | $CH(CH_3)_2$ |
| 4 | H | H | $CH_2CH_2CH_2CH_3$ |
| 5 | H | H | $CH(CH_3)CH_2CH_3$ |
| 6 | H | H | $C(CH_3)_3$ |
| 7 | 4-$CH_3$ | H | $CH_3$ |
| 8 | 4-$CH_3$ | H | $C_2H_5$ |
| 9 | 4-$CH_3$ | H | $CH(CH_3)_2$ |
| 10 | 4-$CH_3$ | H | $CH_2CH_2CH_2CH_3$ |
| 11 | 4-$CH_3$ | H | $CH(CH_3)CH_2CH_3$ |
| 12 | 4-$CH_3$ | H | $C(CH_3)_3$ |
| 13 | 2-F | H | $CH_3$ |
| 14 | 2-F | H | $C_2H_5$ |
| 15 | 2-F | H | $CH(CH_3)_2$ |
| 16 | 2-F | H | $CH_2CH_2CH_2CH_3$ |
| 17 | 2-F | H | $CH(CH_3)CH_2CH_3$ |
| 18 | 2-F | H | $C(CH_3)_3$ |

Table 1

| Compound No. | $(R^1)_m$ | $(R^2)_n$ | $R^3$ |
|---|---|---|---|
| 19 | 2-F, 4-$CH_3$ | H | $CH_3$ |
| 20 | 2-F, 4-$CH_3$ | H | $CH(CH_3)_2$ |
| 21 | 2-F, 4-$CH_3$ | H | $C(CH_3)_3$ |
| 22 | 2-F, 6-F | H | $CH(CH_3)_2$ |
| 23 | 2-F, 6-F | H | $C(CH_3)_3$ |
| 24 | 2-F, 4-$CH_3$, 6-F | H | $CH(CH_3)_2$ |
| 25 | 2-F, 4-$CH_3$, 6-F | H | $C(CH_3)_3$ |
| 26 | 4-$CH_3$ | H | $OC_2H_5$ |
| 27 | 4-$CH_3$ | H | $OCH(CH_3)_2$ |
| 28 | 4-$CH_3$ | H | $OCH_2CH_2CH_2CH_2CH_3$ |
| 29 | H | H | Cl |
| 30 | H | H | Br |
| 31 | 4-$CH_3$ | H | Cl |
| 32 | 4-$CH_3$ | H | Br |
| 33 | 2-F | H | Cl |
| 34 | 2-F, 4-$CH_3$ | H | Cl |
| 35 | H | 3-Cl | Cl |
| 36 | 4-$CH_3$ | 3-Cl | Cl |

**Table 1**

| Compound No. | $(R^1)_m$ | $(R^2)_n$ | $R^3$ |
|---|---|---|---|
| 37 | H | H | $CF_3$ |
| 38 | 2-F | H | $CF_3$ |
| 39 | $3\text{-}CH_3$ | H | $CF_3$ |
| 40 | $4\text{-}CH_3$ | H | $CF_3$ |
| 41 | 2-F, $4\text{-}CH_3$ | H | $CF_3$ |
| 42 | 2-F, 6-F | H | $CF_3$ |
| 43 | H | H | O- 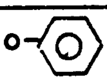 |
| 44 | H | H | 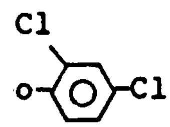 |
| 45 | $4\text{-}CH_3$ | H | O- 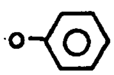 |
| 46 | 2-F | H | O- 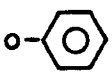 |
| 47 | H | H | $OCH_2$- 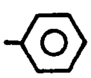 |
| 48 | $4\text{-}CH_3$ | H | $OCH_2$- 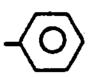 |
| 49 | 2-F | H | $OCH_2$- 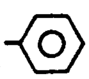 |
| 50 | H | H | $CH_2$- 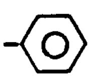 |
| 51 | $4\text{-}CH_3$ | H | $CH_2$- 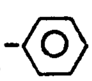 |

Table 1

| Compound No. | $(R^1)_m$ | $(R^2)_n$ | $R^3$ |
|---|---|---|---|
| 52 | H | H | 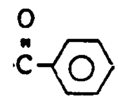 |
| 53 | 4-$CH_3$ | H | 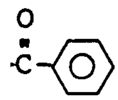 |
| 54 | 2-F | H | 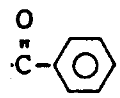 |
| 55 | H | 3-F | $-CF_3$ |
| 56 | 4-$CH_3$ | 3-F | $-CF_3$ |
| 57 | 2-F | 3-F | $-CF_3$ |
| 58 | H | 3-F | $C(CH_3)_3$ |
| 59 | 4-$CH_3$ | 3-F | $-C(CH_3)_3$ |
| 60 | 2-F | 3-F | $-C(CH_3)_3$ |
| 61 | H | 3-F | 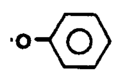 |
| 62 | 4-$CH_3$ | 3-F | 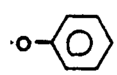 |
| 63 | 2-F | 3-F | 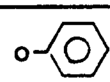 |
| 64 | H | H | $-\overset{O}{\overset{\|}{C}}-CH_3$ |
| 65 | 4-$CH_3$ | H | $-\overset{O}{\overset{\|}{C}}-CH_3$ |

Table 1

| Compound No. | $(R^1)_m$ | $(R^2)_n$ | $R^3$ |
|---|---|---|---|
| 66 | H | H | 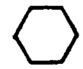 |
| 67 | 4-$CH_3$ | H | 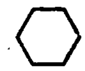 |
| 68 | H | 3-$CH_3$ | $CH_3$ |
| 69 | H | 3-Cl,5-Cl | $CH_3$ |

Examples of the salts of the compound of general formula (I) include inorganic acid salts such as hydrochlorides, hydrobromides, hydroiodides, hydrofluorides, sulfates, hydrogensulfates, nitrates, chlorates, perchlorates, phosphates, hydrogenphosphates, dihydrogenphosphates, thiocyanates and tetrafluoroborates, and organic acid salts such as formates, acetates, trichloroacetates, trifluoroacetates, citrates, lactates, oxalates, glycollates, malonates, succinates, malates, dodecylbenzenesulfonates, benzoates, salicylates and nicotinates.

The compound of general formula (I) can be produced by the following process.

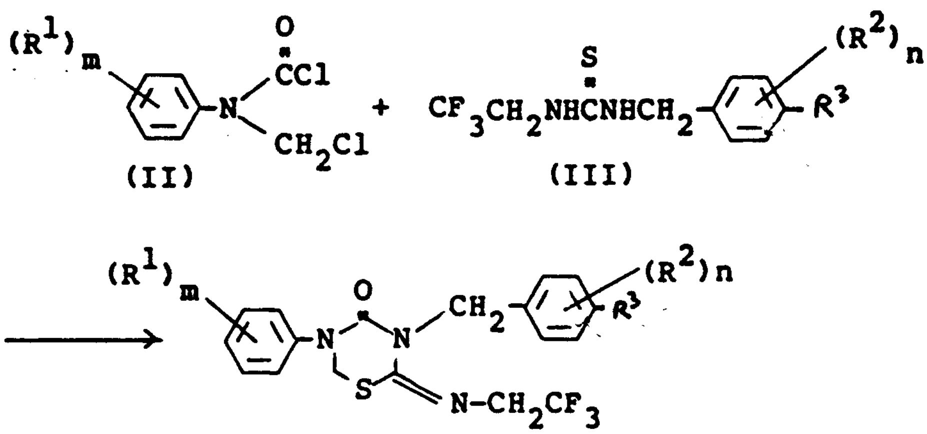

(II) (III)

(I)

In the formulae, R¹, R², R³, m and n are as defined above.

Specifically, the compound of general formula (I) can be obtained by reacting the carbamoyl chloride derivative of general formula (II) with the thiourea derivative of general formula (III) in the presence or absence of a solvent, preferably in the presence of a solvent. Examples of suitable solvents include acetone, methyl ethyl ketone, cyclohexanone, tetrahydrofuran, dioxane, ethyl ether, benzene, toluene, acetonitrile, ethanol, propanol, dichloromethane, chloroform, carbon tetrachloride, dimethylformamide, dimethylacetamide, dimethyl sulfoxide, 1,3-dimethyl-2-imidazolidinone and water. Other solvents which do not affect the reaction may also be used.

The reaction is carried out by heating or in the presence of a base. When the reaction is carried out by heating, the reaction temperature may be varied over a broad range depending upon the starting material. Generally, the reaction is carried out at a temperature of 30 to 250 °C, preferably 40 to 150 °C, for a period of 0.1 to 30 hours, preferably 0.5 to 24 hours. Examples of suitable bases which may be used in the reaction include potassium hydroxide, sodium hydroxide, potassium carbonate, sodium carbonate, sodium hydrogen carbonate, triethylamine, pyridine. N,N-dimethylaniline, and 1,8-diazabicyclo-[5,4,0]-7-undecene.

The reaction temperature and the reaction time may be varied over wide ranges depending upon the starting materials used. Generally, the reaction conducted in the presence of the base is carried out at a temperature of -10 to 200 °C, preferably room temperature to 150 °C, for a period of 0.1 to 30 hours, preferably 0.5 to 24 hours.

In carrying out the above reaction in accordance with this invention, the carbamoyl chloride derivative of general formula (II) and the thiourea derivative of formula (III) may be used in equimolar proportions, or one of them may be used in a slightly excessive molar proportion. When it is desired to obtain the compound of general formula (I) in a free form using the base, it is desirable to use the base in an amount of 2 or slightly more moles per mole of the carbamoyl chloride derivative of general formula (II).

The carbamoyl chloride derivative of general formula (II) used as one starting material in the above reaction may be synthesized by a known method [Journal of Organic Chemistry, vol. 39, page 2897, (1974)]. The thiourea derivative of formula (III) may be synthesized in accordance with a known method.

The salt of the compound of general formula (I) may be produced in accordance with a known method. Specifically, it can be produced by treating the compound of general formula (I) with an inorganic or organic acid. Examples of the acid include inorganic acids such as hydrochloric acid, hydrobromic acid, hydroiodic acid, hydrofluoric acid, sulfuric acid, nitric acid, chloric acid, perchloric acid, phosphoric acid, thiocyanic acid or tetrafluoroboric acid, and organic acids such as formic acid, acetic acid, tetrachloroacetic acid, trifluoroacetic acid, citric acid, lactic acid, oxalic acid, glycollic acid, malonic acid, succinic acid, malic acid, dodecylbenzenesulfonic acid, benzoic acid, salicyclic acid or nicotinic acid.

The compounds of this invention represented by general formula (I) can be used to protect plants from various noxious arthropods encountered in the fields of agriculture, forestry, horticulture, etc.

For example, the compounds of formula (I) are effective against hemipterous insect pests such as small brown planthopper, brown planthopper, whitebacked planthopper, green rice leafhopper, zig-zag rice leafhopper, tea green leafhopper, jumping plantlice, westwood-greenhouse whitefly, citrus spiny whitefly, green peach aphid, cotton aphid, cabbage aphid, spiraea aphid, lace bug, bean bug, cletus punctiger Dallas, rice bug, white-spotted bug, southern green stink bug, arrowhead scale, San jose scale, and white peach scale; lepidopterous insect pests such as rice stem borer, rice leafroller, oriental corn borer, rice skipper, green rice caterpillar, apple leafminer, beet semi-looper, black cutworm, cutworm, summer fruit tortrix, apple leafroller, peach fruit moth, citrus leafminer, pear leafminer, cherry treeborer, gypsy moth, fall webworm, cabbage moth, rice armyworm, cabbage armyworm, tobacco cutworm and common cabbageworm; coleopterous insect pests such as cupreous chafer, soybean beetle, Japanese beetle, citrus flower chafer, rice water weevil, rice plant weevil and sugarcane wireworm; dipterous insect pests such as rice crane fly, soybean pod gall midge, melon fly, oriental fruit fly, rice leafminer, stone leek leafminer, bryony leafminer, onion maggot and seedcorn maggot; and thrips such as yellow tea thrips, Thrips palmi Karny and onion thrips.

They are also effective against pests which cause various injuries to man and domestic animals, for example, the transmission of epidemics, blood sucking, stinging and biting and skin inflammation, such as house mosquito, Culex pipiens molestus, Culex tritaeniorhyncus, Aedes albopictus, house flies, Boettcherisca peregrina Robineau-Desvoidy, Calliphora lata Coquillett, Phormia regina Meigen, Drosophila melanogaster, American cockroach, German cockroach, smokybrown cockroach, Periplaneta brunnea Burmeister, Japanese cockroach, Ornithonyssus bacoti Hirst, human louse, Pediculus humanus humanus De Geer, Climex lectularus Linne, human flea, dog flea, cat flea, oriental tussock moth, tea tussock moth, Scolopendra subspinipes japonica, rove beetle, and Xanthochroa waterhousei Harold; pests which damage foods or stored grains, such as mold mite, bread beetle, confused flour beetle, maize weevil, azuki bean weevil, common hide beetle and Indian meal moth; pests which damage furniture, building materials, books and apparel such as Reticulitermes speratus Kolbe, Formosan subterranean termite, powderpost beetle, Gastrallus immarginatus Mullerr, casemaking clothes moth and black carpet beetle; and so-called "unpleasant pests", such as Telmatoscopus albipunctatus Williston, Chironomus plumosus Linnaeus, midges, camel crickets, brown marmorated stink bug, Thereuronema hilgendorfi Verhoeff, Oxidus gracilis C. L. Koch, pillbug and Porcellio scaber Latreille.

The compounds of this invention show much higher insecticidal activity on lepidopterous insect pests than known compounds.

In actual application, the compound of the invention may be used singly without other components, but to make it easy to use as a control agent, it is generally applied as a mixture with a carrier. Formulation of the compound of the invention requires no particular conditions, and it may be prepared in any desired form such as an emulsifiable concentrate, a wettable powder, a dust, granules, a pulverulent agent, an oil, an aerosol, a fumigant or a bait by methods well known to those skilled in the art in accordance with the formulation of general agricultural chemicals.

The carrier, as used herein, denotes a synthetic or a natural inorganic or organic material which is incorporated in order to aid in the arrival of the active ingredient at a site to be treated or facilitate storage, transportation and handling of the active ingredient compound.

Suitable solid carriers include, for example, clays such as montmorillonite and kaolinite; inorganic materials such as diatomaceous earth, terra alba, talc, vermiculite, gypsum, calcium carbonate, silica gel and ammonium sulfate, organic plant materials such as soybean meal, sawdust and wheat flour; and urea.

Suitable liquid carriers include, for example, aromatic hydrocarbons such as toluene, xylene and cumene, paraffinic hydrocarbons such as kerosene and mineral oils, halogenated hydrocarbons such as carbon tetrachloride, chloroform and dichloroethane, ketones such as acetone and methyl ethyl ketone, ethers such as dioxane and tetrahydrofuran, alcohols such as methanol, ethanol, propanol and ethylene glycol, dimethylformamide, dimethyl sulfoxide, and water.

To enhance the efficacy of the compounds of this invention, various adjuvants, either singly or in combination, may be combined with the compounds of the invention according to the formulation of the compounds, the situation in which they are applied, etc.

For the purpose of emulsification, dispersion, spreading, wetting, binding and stabilization, there may be used anionic surface-active agents such as lignosulfonates, alkylbenzenesulfonates and alkylsulfates; nonionic surface-active agents such as polyoxyalkylene alkyl ethers, polyoxyalkylene alkyl aryl ethers, polyoxyalkylene alkylamines, polyoxyalkylene alkylamides, polyoxyalkylene alkyl thioethers, polyoxyalkylene fatty acid esters, glycerin fatty acid esters, sorbitan fatty acid esters, polyoxyalkylene sorbitan fatty acid esters and polyoxypropylene polyoxyethylene block polymers; lubricants such as calcium stearate and waxes; stabilizers such as isopropyl hydrogen phosphate; and methyl cellulose, carboxymethyl cellulose, casein and gum arabic.

A better insecticidal activity may be obtained by using two or more compounds of this invention in combination. Furthermore, multipurpose compositions having a better efficacy may be prepared by mixing the compounds of the invention with other insecticides or acaricides, fungicides, nematocides, herbicides, plant growth regulating agents, fertilizers, machine oils and other agricultural chemicals. Synergistic effects can be expected from such compositions. Examples of the other insecticides or acaricides include fenthion, fenitrotion, diazinon, chlorpyrifos, chlorpyrifos-methyl, methidathion, dichlorvos, thiometon, acephate, trichlorphon, isoxathion, pyridafenthion, salithion, prothiofos, propaphos, EPN, sulprofos, NAC, MTMC, MIPC, BPMC, PHC, MPMC, XMC, pirimicarb, carbosulfan, benfuracarb, methomyl, oxamyl, pyrethrin, tetramethrin, phthalthrin, vaporthrin, allethrin, resmethrin, fenvalerate, esfenvalerate, permethrin, cypermethrin, fluvalinate, ethofenprox, flucythrinate, cyhalothrin, bifenthrin, diflubenzuron, chlorfluazuron, teflubenzuron, flufenoxuron, cypromazine, buprofezin, fenoxycarb, benzoepin, nereistoxin, bensultap, thiocyclam, avermectin, dicofol, amitraz, polynactins, fenbutatin oxide, cyhexatin, hexythiazox, flubenzamine, triarathene, clofentezine and milbemycin.

The compounds of this invention are stable to light, heat and oxidation. If required, however, suitable amount of stabilizers, for example antioxidants or ultraviolet absorbers such as phenol derivatives [e.g., BHT (2,6-di-t-butyl-4-methylphenol) and BHA (butylhydroxyanisole)], bisphenol derivatives, arylamines (e.g., phenyl-$\alpha$-naphthylamine, phenyl-$\beta$-naphthylamine, or a condensate of phenetidine and acetone), and benzophenone compounds may be added. This can give a composition having a more stabilized efficacy.

In the insecticide of this invention, 0.0001 to 95 % by weight, preferably 0.001 to 50 % by weight, of the compound of the invention is included as an active ingredient. In applying the insecticide of this invention, the active ingredient is desirably used in a concentration of 0.01 to 5000 ppm, preferably 0.1 to 1000 ppm. The ratio of application is generally 1 g to 300 g of the active ingredient per 10 a.

The following examples illustrate the process for producing the compounds of the invention represented by general formula (I).

EXAMPLE 1

Synthesis of 2-(2,2,2-trifluoroethylimino)-3-(4-t-butylbenzyl)-5-phenyl-tetrahydro-1,3,5-thiadiazin-4-one (compound No. 6)

A solution of 0.65 g of trichloromethyl chloroformate in 10 ml of benzene was added dropwise to a solution of 0.69 g of 1,3,5-triphenyl-hexahydro-s-triazine in 20 ml of tetrahydrofuran with stirring at room temperature in a nitrogen stream. The solution was stirred at room temperature for 1.5 hours. Then, 2.00 g of 1-(4-t-butylbenzyl)-3-(2,2,2-trifluoroethyl)thiourea was added at room temperature with stirring, and subsequently, 6 ml of a 10 % aqueous solution of sodium hydroxide was added. The mixture was stirred at room temperature for 12 hours. Water (30 ml) was added to the reaction mixture, and it was extracted with

150 ml of ethyl acetate. The ethyl acetate solution was washed with water, and dried. Ethyl acetate was evaporated under reduced pressure. The resulting oily product was purified by column chromatography [silica gel; eluent: hexane/ethyl acetate (10/1)] to give 0.42 g of the captioned compound.

Melting point: 113.0-114.0 °C.

IR $\nu_{max}^{KBr}$ ($cm^{-1}$): 2960, 1665, 1605, 1490, 1460, 1420, 1400, 1385, 1295, 1275, 1265, 1210, 1170, 1145, 1130, 1120, 1090, 950, 715.

$^1H$ NMR $\delta_{TMS}^{CDCl_3}$ (ppm): 1.33(9H, s), 3.84(2H, q, J=9Hz), 4.87(2H, s), 5.32 (2H, s), 7.2-7.6(9H, m).

As an isomer, 0.37 g of 2-(4-t-butylbenzylimino)-3-(2,2,2-trifluoroethyl)-5-phenyl-tetrahydro-1,3,5-thiadiazin-4-one was obtained.

Melting point: 90.0-91.5 °C.

IR $\nu_{max}^{KBr}$ ($cm^{-1}$): 2960, 1670, 1610, 1490, 1450, 1390, 1365, 1335, 1260, 1200, 1160, 1120, 750.

$^1H$ NMR $\delta_{TMS}^{CDCl_3}$ (ppm): 1.37(9H, s), 4.57(2H, s), 4.92 (2H, s), 5.16(2H, q, J=9Hz), 7.2-7.6(9H, m).

EXAMPLE 2

Synthesis of 2-(2,2,2-trifluoroethylimino)-3-(4-t-butylbenzyl)-5-(4-methylphenyl)-tetrahydro-1,3,5-thiadiazin-4-one (compound No. 12)

A solution of 1.42 g of trichloromethyl chloroformate in 20 ml of benzene was added dropwise to a solution of 1.72 g of 1,3,5-tris(4-methylphenyl)-hexahydro-s-triazine in 30 ml of tetrahydrofuran at room temperature with stirring in a nitrogen stream. The reaction solution was stirred at room temperature for 1 hour. Then, 4,0 g of 1-(4-t-butylbenzyl)-3-(2,2,2-trifluoroethyl)thiourea was added at room temperature with stirring, and subsequently 12 ml of a 10 % aqueous solution of sodium hydroxide was added. The mixture was stirrerd at room temperature for 6 hours. Water (50 ml) was added to the reaction mixture, and it was extracted with 200 ml of ethyl acetate. The ethyl acetate layer was washed with water and dried, and ethyl acetate was evaporated under reduced pressure. The resulting oily product was purified by column chromatography [silica gel; eluent: hexane/ethyl acetate (10/1)] to give 0.79 g of the captioned compound.

Refractive index: $n_D^{20}$=1.5464.

IR $\nu_{max}^{neat}$ ($cm^{-1}$): 2960, 1690, 1615, 1515, 1450, 1395, 1290, 1270, 1210, 1150, 1090, 940, 850, 825, 755, 725.

$^1H$ NMR $\delta_{TMS}^{CDCl_3}$(ppm): 1.34(9H, s), 2.36(3H, s), 3.81 (2H, q, J=9Hz), 4.79(2H, s), 5.27(2H, s), 7.18-7.35(8H, m).

As an isomer, 1.29 g of 2-(4-t-butylbenzylimino)-3-(2,2,2-trifluoroethyl)-5-(4-methylphenyl)tetrahydro-1,3,5-thiadiazin-4-one was obtained.

Refractive index: $n_D^{20}$=1.5537.

IR $\nu_{max}^{neat}$ ($cm^{-1}$): 2960, 1700, 1620, 1515, 1450, 1395, 1335, 1305, 1270, 1210, 1160, 1120, 1080, 1045, 1020, 835, 820, 770.

$^1H$ NMR $\delta_{TMS}^{CDCl_3}$(ppm): 1.36(9H, s), 2.36(3H, s), 4.52 (2H, s), 4.84(2H, s), 5.11(2H, q, J=9Hz), 7.18-7.41(8H. m).

EXAMPLE 3

Synthesis of 2-(2,2,2-trifluoroethylimino)-3-(4-fluoromethylbenzyl)-5-phenyl-tetrahydro-1,3,5-thiadiazin-4-one (compound No. 37)

A solution of 2.5 g of trichloromethyl chloroformate in 10 ml of benzene was added dropwise to a solution of 2.65 g of 1,3,5-triphenyl-hexahydro-s-triazine in 20 ml of tetrahydrofuran at room temperature with stirring in a nitrogen stream. The reaction solution was stirred at room temperature for 1.5 hours. Then, 4.00 g of 1-(4-trifluoromethylbenzyl)-3-(2,2,2-trifluoroethyl)thiourea was added at room temperature with stirring, and subsequently 15 ml of a 10 % aqueous solution of sodium hydroxide was added. The mixture was stirred at room temperature for 12 hours. Water (30 ml) was added to the reaction mixture, and it was extracted with 150 ml of ethyl acetate. The ethyl acetate solution was washed with water and dried. Under reduced pressure, ethyl acetate was evaporated. The resulting oily product was purified by column chromatography [silica gel; eluent: hexane/ethyl acetate (10/1)] to give 1.34 g of the captioned compound as a semisolid.

IR $\nu_{max}^{neat}$ ($cm^{-1}$): 1680, 1615, 1500, 1450, 1390, 1330, 1270, 1140, 1125, 1065, 1015, 935, 840, 755.

$^1H$ NMR $\delta_{TMS}^{CDCl_3}$ (ppm): 3.80(2H, q, J=9Hz), 4.88(2H, s), 5.23(2H, s), 7.18-7.50(8H, m).

As an isomer, 0.76 g of 2-(4-trifluoromethylbenzylimino)-3-(2,2,2-trifluoroethyl)-5-phenyl-tetrahydro-1,3,5-thiadiazin-4-one was obtained.

Melting point: 127.0-128.0 °C.

IR $\nu_{max}^{KBr}$ ($cm^{-1}$): 1695, 1625, 1500, 1455, 1400, 1325, 1280, 1160, 1120, 1070, 1025, 835, 825, 755.

$^1H$ NMR $\delta_{TMS}^{CDCl_3}$ (ppm): 4.60(2H, s), 4.90(2H, s), 5.11 (2H, q, J=9Hz), 7.26-7.44(9H, m).

EXAMPLE 4

Synthesis of 2-(2,2,2-trifluoroethylimino)-3-(4-trifluoromethylbenzyl)-5-(2-fluorophenyl)-tetrahydro-1,3,5-thiadiazin-4-one (compound No. 38)

A solution of 1.00 g of trichloromethyl chloroformate in 20 ml of benzene was added dropwise to a solution of 1.23 g of 1,3,5-tri(2-fluorophenyl)-hexahydro-s-triazine in 30 ml of tetrahydrofuran at room temperatue with stirring in a nitrogen stream. The reaction solution was stirred at room temperature for 1 hour. Then, 3,16 g of 1-(4-trifluoromethylbenzyl)-3-(2,2,2-trifluoroethyl)thiourea was added with stirring at room temperature, and subsequently, 1.5 ml of triethylamine was added. The mixture was stirred at room temperature for 6 hours. Water (50 ml) was added to the reaction mixture, and it was extracted with 200 ml of ethyl acetate. The ethyl acetate solution was washed with water and dried. Under reduced pressure, ethyl acetate was evaporated. The resulting oily product was purified by column chromatography [silica gel; eluent: hexane/ethyl acetate (10/1)] to give 0.20 g of the captioned compound.

Refractive index: $n_D^{20}$=1.5225.

IR $\nu_{max}^{neat}$ ($cm^{-1}$): 1690, 1620, 1505, 1450, 1400, 1330, 1290, 1275, 1265, 1260, 1240, 1150, 1130, 1090, 1070, 1020, 940, 845, 760.

$^1H$ NMR $\delta_{TMS}^{CDCl_3}$ (ppm): 3.72(2H, q, J=9Hz), 4.72 (2H, s), 5.29(2H, s), 7.00-7.50(4H, m), 7.51(4H, s).

As an isomer, 0.30 g of 2-(4-trifluoromethylbenzylimino)-3-(2,2,2-trifluoroethyl)-5-(2-fluorophenyl)-tetrahydro-1,3,5-thiadiazin-4-one was obtained.

Refractive index: $n_D^{20}$=1.5291.
IR $\nu_{max}^{neat}$ ($cm^{-1}$): 1700, 1620, 1505, 1445, 1415, 1400, 1325, 1275, 1260, 1235, 1160, 1125, 1110, 1065, 1020, 830, 760.
$^1H$ NMR $\delta_{TMS}^{CDCl_3}$ (ppm): 4.52(2H, s), 4.77(2H, s), 5.11 (2H, q, J=10Hz), 6.90-7.70 (8H, m).

EXAMPLE 5

Synthesis of 2-(2,2,2-trifluoroethylimino)-3-(4-trifluoromethylbenzyl)-5-(3-methylphenyl)-tetrahydro-1,3,5-thiadiazin-4-one (compound No.39)

A solution of 1.00 g of trichloromethyl chloroformate in 10 ml of benzene was added dropwise to a solution of 1.19 g of 1,3,5-tri(3-methylphenyl)-hexahydro-s-triazine in 20 ml of tetrahydrofuran at room temperature with stirring in a nitrogen stream. The reaction mixture was stirred at room temperature for 1.5 hours. Then, 3.16 g of 1-(4-trifluoromethylbenzyl)-3-(2,2,2-trifluoroethyl)thiourea was added at room temperature with stirring, and subsequently, 4.0 ml of triethylamine was added. The mixture was stirred at room temperature for 12 hours. Aqueous ammonia (30 ml) was added to the reaction mixture, and it was extracted with 150 ml of ethyl acetate. The ethyl acetate solution was washed with water and dried. Under reduced pressure, ethyl acetate was evaporated. The resulting oily product was purified by column chromatography [silica gel; eluent: hexane/ethyl acetate (10/1)] to give 0.54 g of the captioned compound.

Refractive index: $n_D^{20}$=1.5340.
IR $\nu_{max}^{neat}$ ($cm^{-1}$): 1680, 1610, 1490, 1445, 1380, 1315, 1265, 1255, 1105, 1065, 1045, 1025, 935.
$^1H$ NMR $\delta_{TMS}^{CDCl_3}$ (ppm): 2.40(3H, s), 3.82 (2H, q, J=9Hz), 4.86(2H, s), 5.36(2H, s), 7.00-7.50(4H, m), 7.60(4H, s).

As an isomer, 0.31 g of 2-(4-trifluoromethylbenzylimino)-3-(2,2,2-trifluoroethyl)-5-(3-methylphenyl)-tetrahydro-1,3,5-thiadiazin-4-one was obtained.

Melting point: 97.0-98.0 °C.
IR $\nu_{max}^{neat}$ ($cm^{-1}$): 1690, 1615, 1490, 1440, 1415, 1390, 1325, 1270, 1260, 1155, 1110, 1065, 1015.
$^1H$ NMR $\delta_{TMS}^{CDCl_3}$ (ppm): 2.39(3H, s), 4.57(2H, s), 4.88 (2H s), 5.15(2H, q, J=10Hz), 7.00-7.80(8H, m).

EXAMPLE 6

Synthesis of 2-(2,2,2-trifluoroethylimino)-3-(4-trifluoromethylbenzyl)-5-(4-methylphenyl)-tetrahydro-1,3,5-thiadiazin-4-one (compound No. 40)

1.00 g of N-chloromethyl-N-(4-methylphenyl)carbamoyl chloride and 1.45 g of 1-(4-trifluoromethylbenzyl)-3-(2,2,2-trifluoroethyl)thiourea were dissolved in 30 ml of benzene, and the solution was heated under reflux for 4 hours. After the reaction, benzene was evaporated under reduced pressure. The resulting oily product was purified by column chromatography [silica gel; eluent: hexane/ethyl acetate (10/1)] to give 1.27 g of the captioned compound.

Melting point: 62.0-63.0 °C.

IR $\nu_{max}^{KBr}$ ($cm^{-1}$): 1675, 1610, 1510, 1440, 1390, 1325, 1285, 1265, 1205, 1135, 1105, 1060, 1015, 935, 840, 815, 740.

$^1$H NMR $\delta_{TMS}^{CDCl_3}$ (ppm): 2.38(3H, s), 3.78(2H, q, J=9Hz), 4.80(2H, s), 5.35 (2H, s), 7.23(4H, s), 7.57 (4H, s).

As an isomer, 0.30 g of 2-(4-trifluoromethylbenzylimino)-3-(2,2,2-trifluoroethyl)-5-(4-methylphenyl)-tetrahydro-1,3,5-thiadiazin-4-one was obtained.

Melting point: 97.0-98.5 °C.

IR $\nu_{max}^{KBr}$ ($cm^{-1}$): 1675, 1590, 1505, 1440, 1405, 1390, 1410, 1405, 1390, 1315, 1295, 1260, 1150, 1105, 1060.

$^1$H NMR $\delta_{TMS}^{CDCl_3}$ (ppm): 2.40(3H, s), 4.58(2H, s), 4.86 (2H, s), 5.15(2H, q, J=10Hz), 7.24(4H, s), 7.50(2H, d, J=8Hz), 7.65(2H, d, J=8Hz).

EXAMPLE 7

Synthesis of 2-(2,2,2-trifluoroethylimino)-3-(4-isopropyloxybenzyl)-5-(4-methylphenyl)-tetrahydro-1,3,5-thiadiazin-4-one (compound No.27)

1.00 g of N-chloromethyl-N-(4-methylphenyl)carbamoyl chloride and 1.40 g of 1-(4-isopropyloxybenzyl)-3-(2,2,2-trifluoroethyl)thiourea were dissolved in 30 ml of benzene, and the solution was heated under reflux for 4 hours. After the reaction, benzene was evaporated under reduced pressure. The resulting oily product was purified by column chromatography [silica gel; eluent: hexane/ethyl acetate (10/1)] to give 0.75 g of the captioned compound.

Refractive index: $n_D^{20}$=1.5462.

IR $\nu_{max}^{neat}$ ($cm^{-1}$): 1690, 1610, 1510, 1445, 1380, 1260, 1240, 1135, 1080, 1035, 950, 935, 840, 815.

$^1H$ NMR $\delta_{TMS}^{CDCl_3}$(ppm): 1.31(6H, d, J=6Hz), 2.36(3H, s), 3.81(2H, q, J=9Hz), 4.39-4.62 (1H, m), 4.75(2H, s), 5.26 (2H, s), 6.80(2H, d, J=8Hz), 7.18(4H, s), 7.39(2H, d, J=8Hz).

As an isomer, 0.6 g of 2-(4-isopropyloxybenzylimino)-3-(2,2,2-trifluoroethyl)-5-(4-methylphenyl)-tetrahydro-1,3,5-thiaziazin-4-one was obtained.

Melting point: 110.0-112.5 °C.

IR $\nu_{max}^{neat}$ ($cm^{-1}$): 1690, 1610, 1510, 1440, 1385, 1260, 1240, 1150, 1110, 1070, 1040, 1015, 950, 820.

$^1H$ NMR $\delta_{TMS}^{CDCl_3}$(ppm): 1.36(6H, d, J=6Hz), 2.37(3H, s), 4.44(2H, s), 4.38-4.61(1H, m), 4.80(2H, s), 5.10(2H, q, J=10Hz), 6.86(2H, d, J=8Hz), 7.18(4H, s), 7.23(2H, d, J=8Hz).

EXAMPLE 8

Synthesis of 2-(2,2,2-trifluoroethylimino)-3-(3,4-dichlorobenzyl)-5-phenyl-tetrahydro-1,3,5-thiadiazin-4-one (compound No. 35)

1.00 g of N-chloromethyl-N-phenylcarbamoyl chloride and 1.55 g of 1-(3,4-dichlorobenzyl)-3-(2,2,2-trifluoroethyl)thiourea were dissolved in 30 ml of benzene, and the solution was heated under reflux. After the reaction, benzene was evaporated under reduced pressure. The resulting oily product was purified by column chromatography [silica gel; eluent: hexane/ethyl acetate (10/1)] to give 0.88 g of the captioned compound.

Melting point: 99.1-99.6 °C.

IR $\nu_{max}^{KBr}(cm^{-1})$: 1675, 1665, 1610, 1390, 1285, 1265, 1135, 1120, 1085, 1030, 990, 930, 750, 720, 690.

$^1H$ NMR $\delta_{TMS}^{CDCl_3}$(ppm): 3.80(2H, q, J=9Hz), 4.88(2H, s), 5.20(2H, s), 7.27-7.61(8H, m).

As an isomer, 0.20 g of 2-(3,4-dichlorobenzylimino)-3-(2,2,2-trifluoroethyl)-5-phenyl-tetrahydro-1,3,5-thiadiazin-4-one was obtained as a semisolid.

1,3,5-thiadiazin-4-one was obtained as a semisolid.

IR $\nu_{max}^{neat}(cm^{-1})$: 1695, 1620, 1505, 1495, 1470, 1445, 1390, 1330, 1265, 1210, 1155, 1115, 1080, 1030, 835, 815, 760.

$^1H$ NMR $\delta_{TMS}^{CDCl_3}$(ppm): 4.48(2H, s), 4.90(2H, s), 5.08 (2H, q, J=10Hz), 7.10-7.41 (8H, m).

EXAMPLE 9

Synthesis of 2-(2,2,2-trifluoroethylimino)-3-(4-trifluoromethylbenzyl)-5-(2-fluoro-4-methylphenyl)tetrahydro-1,3,5-thiadiazin-4-one (compound No. 41)

0.60 g of N-chloromethyl-N-(2-fluoro-4-methylphenyl)carbamoyl chloride and 0.80 g of 1-(4-trifluoromethylbenzyl)-3-(2,2,2-trifluoroethyl)thiourea were dissolved in 30 ml of toluene, and the solution was heated under reflux for 4 hours. After the reaction, toluene was evaporated under reduced pressure. The resulting oily product was purified by column chromatography [silica gel; eluent: hexane/ethyl acetate (10/1)] to give 0.50 g of the captioned compound as a semisolid.

IR $\nu_{max}^{neat}(cm^{-1})$: 1690, 1620, 1515, 1450, 1400, 1330, 1270, 1140, 1090, 1070, 1020, 940, 850, 825, 765, 750, 725, 710.

$^1H$ NMR $\delta_{TMS}^{CDCl_3}$(ppm): 2.35(3H, s), 3.78(2H, q, J=8Hz), 4.74(2H, s), 5.30 (2H, s), 6.96-7.33(3H, m), 7.61(4H, s).

As an isomer, 0.17 g of 2-(4-trifluoromethylbenzylimino)-3-(2,2,2-trifluoroethyl)-5-(2-fluoro-4-methylphenyl)-tetrahydro-1,3,5-thiadiazin-4-one was obtained.

Melting point: 115-116 °C.

IR $\nu_{max}^{KBr}$ ($cm^{-1}$): 1690, 1630, 1515, 1450, 1400, 1330, 1310, 1255, 1170, 1110, 1070, 1045, 1015, 945, 830, 825, 765, 745.

$^1H$ NMR $\delta_{TMS}^{CDCl_3}$ (ppm): 2.36(3H, s), 4.55(2H, s), 4.77(2H, s), 5.10(2H, q, J=10Hz), 6.96-7.36(3H, m), 7.50(2H, d, J=9Hz), 7.70(2H, d, J=9Hz).

EXAMPLE 10

Synthesis of 2-(2,2,2-trifluoroethylimino)-3-(4-phenoxybenzyl)-5-phenyl-tetrahydro-1,3,5-thiadiazin-4-one (compound No. 43)

1.00 g of N-chloromethyl-N-phenylcarbamoyl chloride and 1.67 g of 1-(4-phenoxybenzyl)-3-(2,2,2-trifluoroethyl)thiourea were dissolved in 30 ml of benzene, and the solution was heated under reflux for 4 hours. After the reaction, benzene was evaporated under reduced pressure. The resulting oily product was purified by column chromatography [silica gel; eluent: solvent: hexane/ethyl acetate (10/1)] to give 0.87 g of the captioned compound.

Refractive index: $n_D^{20}$=1.5903.

IR $\nu_{max}^{neat}$ ($cm^{-1}$): 1680, 1610, 1590, 1500, 1490, 1445, 1390, 1285, 1270, 1265, 1230, 1205, 1165, 1140, 1105, 1090.

$^1H$ NMR $\delta_{TMS}^{CDCl_3}$ (ppm): 3.81(2H, q, J=8Hz), 4.81 (2H, s), 5.23(2H, s), 6.80-7.50 (14H, m).

As an isomer, 0.15 g of 2-(4-phenoxybenzylimino)-3-(2,2,2-trifluoroethyl)-5-phenyl-tetrahydro-1,3,5-thiadiazin-4-one was obtained.

Refractive index: $n_D^{20}$=1.5845

IR $\nu_{max}^{neat}$ ($cm^{-1}$): 1700, 1620, 1590, 1505, 1495, 1445, 1390, 1335, 1305, 1275, 1265, 1240, 1205, 1160, 1120.

$^1H$ NMR $\delta_{TMS}^{CDCl_3}$ (ppm): 4.49(2H, s), 4.85(2H, s), 5.08(2H, q, J=9Hz), 6.80-7.60 (14H, m).

Table 2 below shows the $^{1}H$ NMR data, IR data and physical properties of compounds produced in accordance with the methods of Examples 1 to 10.

Table 2

| Compound No. | $^{1}H$ NMR $\delta^{CDCl_3}_{TMS}$ (ppm) | IR $\nu_{max}$ ($cm^{-1}$) | Melting point or refractive index |
|---|---|---|---|
| 1 | 2.28(3H, s), 3.67(2H, q, J=9Hz), 4.65(2H, s), 5.18(2H, s), 6.90-7.26(9H, m) <$CDCl_3$> | 1680, 1610, 1500, 1450, 1390, 1360, 1270, 1205, 1140, 1090, 1040, 935, 845, 755, 715, 690 <neat> | Semisolid |
| 2 | 1.23(3H, t, J=7Hz), 2.65 (2H, q, J=7Hz), 3.83(2H, q, J=8Hz), 4.80 (2H, s), 5.30(2H, s), 6.70-7.50 (9H, m) <$CDCl_3$> | 1685, 1610, 1500, 1445, 1390, 1345, 1265, 1150, 1090, 1035, 970, 940, 845, 825, 775, 755 <neat> | $n_D^{20}$ 1.5726 |
| 3 | 1.24(6H, d, J=8Hz), 2.68-3.07 (1H, m), 3.76(2H, q, J=9Hz), 4.80(2H, s), 5.32(2H, s), 7.12-7.44(9H, m) <$CDCl_3$> | 1680, 1610, 1490, 1440, 1390, 1270, 1260, 1200, 1140, 1090, 1055, 1040, 940, 890, 845, 760 <neat> | Semisolid |
| 4 | 0.80-1.00(3H, m), 1.10-1.70 (4H, m), 2.58(2H, t, J=7Hz), 3.76(2H, q, J=8Hz), 4.80(2H, s), 5.30(2H, s), 6.60-7.40(9H, m) <$CDCl_3$> | 1680, 1610, 1495, 1440, 1380, 1340, 1260, 1200, 1140, 1090, 1035, 935, 840, 750 <neat> | $n_D^{20}$ 1.5501 |

Table 2

| Compound No. | $^1H$ NMR $\delta^{CDCl_3}_{TMS}$ (ppm) | IR $\nu_{max}$ ($cm^{-1}$) | Melting point or refractive index |
|---|---|---|---|
| 5 | 0.80(3H, t, J=6Hz), 1.12-1.24 (3H, m), 1.42-1.68(2H, m), 2.34-2.62(1H, m), 3.75(2H, q, J=9Hz), 4.75(2H, s), 5.16 (2H, s), 6.94-7.25(9H, m) <$CDCl_3$> | 1680, 1625, 1550, 1500, 1450, 1390, 1280, 1265, 1200, 1140, 1090, 1040, 935, 845, 755 <neat> | Semisolid |
| 7 | 2.30(3H, s), 2.37(3H, s), 3.80 (2H, q, J=8Hz), 4.77(2H, s), 5.32(2H, s), 7.00-7.50(8H, m) <$CDCl_3$> | 1690, 1610, 1510, 1445, 1390, 1290, 1265, 1205, 1140, 1090, 1040, 1020, 940, 740, 705 <neat> | Semisolid |
| 9 | 1.20(3H, s), 1.33(3H, s), 2.40 (3H, s), 2.87(1H, d, q, J=7Hz, J=5Hz), 3.87(2H, q, J=8Hz), 4.86(2H, s), 5.33(2H, s), 7.20 (2H, d, J=6Hz), 7.23(4H, s), 7.50(2H, d, J=6Hz) <$CDCl_3$> | 1670, 1610, 1510, 1455, 1395, 1365, 1280, 1265, 1210, 1145, 1125, 1085, 1040, 940, 880, 845, 820, 755, 720 <KBr> | 84.5-86.0 °C |
| 18 | 1.32(9H, s), 3.80(2H, q, J=9Hz), 4.73(2H, s), 5.27(2H, s), 7.00-7.50(8H, m) <$CDCl_3$> | 1680, 1610, 1515, 1500, 1460, 1440, 1400, 1385, 1360, 1270, 1260, 1255, 1235, 1130, 1090, 1040, 1020, 940 <neat> | $n_D^{20}$ 1.5455 |

## Table 2

| Compound No. | $^1H$ NMR $\delta^{CDCl_3}_{TMS}$ (ppm) | IR $\nu_{max}$ ($cm^{-1}$) | Melting point or refractive index |
|---|---|---|---|
| 21 | 1.26(9H, s), 2.33(3H, s), 3.76 (2H, q, J=9Hz), 4.64(2H, s), 5.22(2H, s), 6.88-7.43(7H, m) <$CDCl_3$> | 1690, 1620, 1520, 1450, 1400, 1370, 1290, 1275, 1210, 1150, 1090, 940, 850, 820, 760, 730 710 <neat> | 114-116 °C |
| 26 | 1.35(3H, t, J=8Hz), 2.30(3H, s), 3.74(2H, q, J=8Hz), 3.93(2H, q, J=8Hz), 4.62(2H, s), 4.14(2H, s), 6.72(2H, d, J=8Hz), 7.10(4H, s), 7.29(2H, d, J=8Hz) <$CDCl_3$> | 1680, 1610, 1515, 1445, 1390, 1290, 1270, 1245, 1205, 1175, 1140, 1085, 1045, 935, 845, 815, 740 <neat> | $n_D^{20}$ 1.5535 |
| 28 | 0.84-0.99(3H, m), 1.25-1.50 (4H, m), 1.60-1.88(2H, m), 2.36 (3H, s), 3.74(2H, t, J=9Hz), 3.87(2H, q, J=7Hz), 4.72(2H, s), 5.24(2H, s), 6.80(2H, d, J=8Hz), 7.18(4H, s), 7.43(2H, d, J=8Hz) <$CDCl_3$> | 1670, 1610, 1460, 1440, 1400, 1320, 1270, 1150, 1100, 1060, 1010, 970, 885, 825, 810, 760 <neat> | $n_D^{20}$ 1.5397 |
| 29 | 3.41(2H, q, J=9Hz), 4.74(2H, s), 5.20(2H, s), 7.00-7.50(9H, m) <$CDCl_3$> | 1685, 1620, 1505, 1495, 1450, 1390, 1365, 1290, 1280, 1270, 1210, 1150, 1095 | $n_D^{20}$ 1.5737 |

Table 2

| Compound No. | $^1H$ NMR $\delta_{TMS}^{CDCl_3}$ (ppm) | IR $\nu_{max}$ ($cm^{-1}$) | Melting point or refractive index |
|---|---|---|---|
| 30 | 3.73(2H, q, J=9Hz), 4.77(2H, s), 5.14(2H, s), 7.00-7.50(9H, m) <$CDCl_3$> | 1680, 1615, 1500, 1490, 1450, 1390, 1275, 1210, 1150, 1130, 1110, 1090, 1070, 1045, 1015, 940 <neat> | $n_D^{20}$ 1.5732 |
| 31 | 2.38(3H, s), 3.77(2H, q, J=9Hz), 4.75(2H, s), 5.24(2H, s), 7.14-7.46(8H, m) <$CDCl_3$> | 1680, 1610, 1510, 1490, 1450, 1380, 1350, 1270, 1260, 1210, 1160, 1090, 1040, 1015, 935, 890, 850, 820, 800 <neat> | Semisolid |
| 36 | 2.34(3H, s), 3.74(2H, q, J=10Hz), 4.76(2H, s), 5.11(2H, s), 7.12-7.50(7H, m) <$CDCl_3$> | 1690, 1675, 1650, 1615, 1520, 1475, 1400, 1270, 1150, 1125, 1095, 1035, 935, 825, 760, 740, 690, 670 <KBr> | 108.6-110.1°C |
| 44 | 3.78(2H, q, J=9Hz), 4.80(2H, s), 5.23(2H, s), 6.79-7.56(12H, m) <$CDCl_3$> | 1680, 1610, 1505, 1445, 1390, 1260, 1140, 1095, 1040, 935, 840, 690 <neat> | Semisolid |

Table 2

| Compound No. | $^1H$ NMR $\delta^{CDCl_3}_{TMS}$ (ppm) | IR $\nu_{max}$ ($cm^{-1}$) | Melting point or refractive index |
|---|---|---|---|
| 47 | 3.88(2H, q, J=9Hz), 4.89(2H, s), 5.11(2H, s), 5.31(2H, s), 6.98 (2H, d, J=9Hz), 7.10-7.60 (12H, m) <$CDCl_3$> | 1675, 1610, 1505, 1450, 1385, 1270, 1260, 1140, 1090, 1040, 1025 <neat> | $n_D^{20}$ 1.5892 |
| 48 | 2.36(2H, s), 3.89(2H, q, J=9Hz), 4.72(2H, s), 5.04(2H, s), 5.27(2H, s), 6.80-7.56(13H, m) <$CDCl_3$> | 1680, 1610, 1515, 1455, 1390, 1270, 1145, 1090, 940, 820, 750, 695 <neat> | $n_D^{20}$ 1.5720 |

The following Referential Examples show the production of the starting materials used in the process of this Invention.

REFERENTIAL EXAMPLE 1

Synthesis of 1-(4-t-butylbenzyl)-3-(2,2,2-trifluoroethyl)thiourea

(1) With stirring, 8.16 g of 4-t-butylbenzylamine was added dropwise at -10 °C to a mixture of 10.32 g of dichlorohexyl carbodiimide (DCC), 20 ml of carbon disulfide and 100 ml of ethyl ether. The temperature was returned to room temperature, and the mixture was left to stand for 12 hours. The reaction mixture was filtered, and the residue was washed with ethyl ether. The filtrate and the washing were combined, and the solvent was evaporated under reduced pressure. The resulting oily product was purified by column chromatography [silica gel; eluent: hexane/ethyl acetate (19/1)] to give 8.41 g of 4-t-butylbenzyl isothiocyanate.

Melting point: 44.0-47.0 °C

IR $\nu_{max}^{KBr}$ ($cm^{-1}$): 2940, 2850, 2170, 2100, 1510, 1460, 1410, 1340, 1305, 1280, 1270, 1210, 1200, 1110, 1085, 1020, 805, 710, 540.

$^1H$ NMR $\delta_{TMS}^{CDCl_3}$ (ppm): 1.34(9H, s), 4.64(2H, s), 7.22 (2H, d, J=8Hz), 7.37(2H, d, J=8Hz).

Reference: Angewandte Chemie International Edition: vol. 6, page 174 (1967).

(2) The 4-t-butylbenzyl isothiocyanate (10.82 g) obtained in (1) above and 7.54 g of 2,2,2-trifluoroethylamine were dissolved in 20 ml of ethyl acetate, and the solution was left to stand at room temperature for 24 hours. Ethyl acetate was evaporated under reduced pressure. The resulting oily product was purified by column chromatography [silica gel; eluent: hexane/ethyl acetate (4/1)] to give 15.80 g of the captioned compound.

Melting point: 101.0-103.0 °C.

IR $\nu_{max}^{neat}$ ($cm^{-1}$): 3260, 3085, 2950, 1570, 1380, 1350, 1320, 1300, 1290, 1255, 1155, 1125, 1050, 970, 935, 830.

$^1H$ NMR $\delta_{TMS}^{CDCl_3}$ (ppm): 1.34(9H, s), 4.30(2H, m), 4.5-4.7(2H, m), 5.8-6.2(1H, br), 6.4-6.8(1H, br), 7.23(2H, d, J=9Hz), 7.37(2H, d, J=9Hz).

REFERENTIAL EXAMPLE 2

Synthesis of 1-(4-trifluoromethylbenzyl)-3-(2,2,2-trifluoroethyl)thiourea

(1) Ten grams of 4-trifluoromethylbenzylamine was added dropwise at -10 °C to a mixture of 11.78 g of dicyclohexylcarbodiimide (DCC), 25 ml of carbon disulfide and 50 ml of ethyl ether. The temperature was then returned to room temperature, and the mixture was left to stand for 12 hours. The reaction mixture was filtered, and the residue was washed with ethyl ether. The filtrate and the washing were combined, and the solvent was evaporated under reduced pressure. The resulting oily product was purified by column chromatography [silica gel; eluent: hexane/ethyl acetate (10/1)] to give 11.15 g of 4-tr

Refractive index: $n_D^{20}$=1.5270.

IR $\nu_{max}^{neat}$ ($cm^{-1}$): 2930, 2180, 2090, 1690, 1620, 1420, 1325, 1165, 1125, 1065, 1020, 815.

$^1H$ NMR $\delta_{TMS}^{CDCl_3}$ (ppm): 4.74(2H, s), 7.47(2H, d, J=8Hz), 7.66(2H, d, J=8Hz).

ifluoromethylbenzyl isothiocyanate.

(2) 7.0 g of the 4-trifluoromethylbenzyl isothiocyanate obtained in (1) above and 3.83 g of 2,2,2-trifluoroethylamine were dissolved in 50 ml of ethyl acetate, and the solution was left to stand at room temperature for 24 hours. Ethyl acetate was evaporated under reduced pressure. The resulting white crystals were recrystallized from hexane to give 8.79 g of the captioned compound.

Melting point: 80.0-82.0 °C.

IR $\nu_{max}^{KBr}$ ($cm^{-1}$): 3230, 3070, 1615, 1570, 1550, 1390, 1380, 1345, 1320, 1305, 1280, 1245, 1160, 1115, 1105, 1060.

$^1H$ NMR $\delta_{TMS}^{CDCl_3}$ (ppm): 4.08-4.40(2H, m), 4.76(2H, d, J=6Hz), 6.02(1H, m), 6.56(1H, m), 7.38(2H, d, J=8Hz), 7.57(2H, d, J=8Hz).

Table 3 shows the $^1H$ NMR data, IR data and physical properties of compounds of general formula (III) produced in accordance with the methods of Referential Examples 1 and 2.

Table 3

| $(R^2)_n$ | $R^3$ | $^1H$ NMR $\delta_{TMS}$ (ppm) | IR $\nu_{max}$ ($cm^{-1}$) | Melting point or refractive index |
|---|---|---|---|---|
| H | 4-$CH_3$ | | 3220, 3050, 1565, 1410, 1370, 1330, 1240, 1145, 1100, 1035, 960, 910, 815, 790 <KBr> | 132.5-133.4°C |
| H | 4-Cl | 4.43(2H, dq, J=6Hz, J=9Hz), 4.78(2H, d, J=9Hz), 7.39 (4H, s) <acetone-$d_6$> | 3260, 3220, 3050, 1565, 1485, 1410, 1370, 1335, 1300, 1245, 1180, 1160, 1105, 1085, 970, 805 <KBr> | 135.0-138.0°C |
| 3-Cl | 4-Cl | 4.09-4.43(2H, m), 4.57(2H, d, J=6Hz), 6.36-6.49(1H, br), 6.79-6.92(1H, br), 6.99-7.37 (4H, m) <$CDCl_3$> | 3290, 3140, 1590, 1490, 1415, 1390, 1350, 1325, 1270, 1200, 1185, 1130, 1050, 990, 985, 885, 845, 830, 740 <KBr> | 78.0-79.7°C |
| H | 4-O- 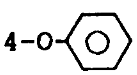 | 4.26(2H, dq, J=6.9Hz), 4.51 (2H, d, J=6Hz), 6.00(1H, brs), 6.58(1H, brs), 6.80-7.40 (9H, m) <$CDCl_3$> | 3240, 1585, 1550, 1530, 1500, 1480, 1370, 1350, 1245, 1160, 1145, 1115, 1100 <KBr> | 126.0-127.5°C |

Table 3

| $(R^2)_n$ | $R^3$ | $^1H$ NMR $\delta_{TMS}$ (ppm) | IR $\nu_{max}$ ($cm^{-1}$) | Melting point or refractive index |
|---|---|---|---|---|
| H | $4-OCH_2-$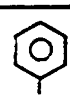 | 1.00-1.90(2H, br), 4.98(2H, dq, J=6Hz, J=9Hz), 4.77(2H, d, J=5Hz), 5.13(2H, s), 6.92-7.60(9H, m) <$CDCl_3$> | 3250, 1610, 1570, 1510, 1380, 1350, 1315, 1295, 1255, 1220, 1160, 1150, 1110, 1020, 690 <KBr> | 118.5-122.0°C |

The following Formulation Examples illustrate agents comprising the compounds of general formula (I) in accordance with this invention as active ingredients and the method of producing them. The invention, however, is not limited to these examples.

FORMULATION EXAMPLE 1

Emulsifiable concentrate:-

| | |
|---|---|
| Compound of the invention | 10 parts |
| Sorpol® 355[s] (tradename for a surface-active agent made by Toho Chemical Co., Ltd.) | 10 parts |
| Xylene | 80 parts |

The above ingredients were mixed to form an emulsifiable concentrate.

FORMULATION EXAMPLE 2

Wettable powder:-

| | |
|---|---|
| Compound of the invention | 20 parts |
| Sodium lignosulfonate | 10 parts |
| Sodium alkylnaphthalenesulfonate | 5 parts |
| White carbon | 5 parts |
| Diatomaceous earth | 60 parts |

The above ingredients were mixed and pulverized uniformly to form a wettable powder.

FORMULATION EXAMPLE 3

Dust:-

Three parts of a compound in accordance with this invention was dissolved in 10 parts of acetone, and 87 parts of clay was added. Acetone was then evaporated to give a dust.

FORMULATION EXAMPLE 4

Granules:-

Three parts of a compound in accordance with the invention, 1 part of sodium lignosulfonate, 20 parts of talc and 76 parts of bentonite were mixed, and kneaded with a moderate amount of water. The mixture was granulated and dried to give granules.

FORMULATION EXAMPLE 5

Bait:-

One part of a compound in accordance with this invention, 5 parts of sugar, 50 parts of wheat bran, 20 parts of rice bran and 24 parts of wheat flour were mixed and kneaded with a moderate amount of water. The mixture was then granulated and dried to give a bait.

The following Test Examples illustrate the superior insecticidal activity of the compounds of this invention. All tests were conducted through three replicates, and the results were shown by averages of the results obtained.

TEST EXAMPLE 1

Effect against tobacco cutworm:-

An emulsifiable concentrate prepared in accordance with Formulation Example 1 was diluted with water to a concentration of 500 ppm and 50 ppm. Sweet potato leaves were immersed in the emulsions, and then air-dried. The treated leaves were transferred to a plastic cup and ten 2nd-instar larvae of tobacco cutworm were caused to feed on the treated leaves for 72 hours. Five days later, the ratio of killing the insects was

examined. The results are shown in Table 4.

It is seen from Table 4 that the compounds of this invention have stronger insecticidal activity than known comparative compounds of a similar structure.

TEST EXAMPLE 2

Effect against cabbage moth:-

An emulsifiable concentrate prepared in accordance with Formulation Example 1 was diluted with water to a concentration of 500 ppm and 50 ppm, and sprayed by a hand sprayer onto cabbage seedlings (5- to 6-leaf stage) in pots to such an extent that the chemical lightly trickled over the leaves. After air drying, the leaves were cut off and put in a plastic cup. Ten 2nd-instar larvae of cabbage moth were caused to feed on the treated leaves for 72 hours, and five days later, the mortality of the insects was examined. The results are shown in Table 5.

Table 5 shows that the compounds of this invention have stronger insecticidal activity than known comparative compounds of a similar structure.

TEXT EXAMPLE 3

Effect against small brown planthopper:-

An emulsifiable concentrate prepared in accordance with Formulation Example 1 was diluted with water to a concentration of 500 ppm and 50 ppm, and sprayed by a hand sprayer onto 5 to 6 rice seedlings (3-leaf stage) to such an extent that the chemical lightly trickled over the seedlings. After air drying, the rice seedlings were held in a plastic cylinder. Ten last-instar larvae of small brown planthopper about one day after ecdysis were inoculated in the rice seedlings. The cylinder was maintained at 25 °C for 16 hours under bright conditions and for 8 hours under dark conditions. Five days later, the mortality of the insects was examined. The results are shown in Table 6.

Table 6 shows that the compounds of this invention have equivalent or stronger insecticidal activity to or than the known comparative compounds of a similar structure.

Thus, the above Test Examples demonstrate that the compounds of this invention have stronger insecticidal activity on lepidopterous insect pests than the known comparative compounds of a similar structure, and equivalent or stronger insecticidal activity on hemipterous insect pests to or than the known comparative comounds of a similar structure.

Table 4

| Test compound No. | Mortality (%) | |
|---|---|---|
| | 500 ppm | 50 ppm |
| 6 | 100 | 100 |
| 9 | 100 | 100 |
| 12 | 100 | 100 |
| 18 | 100 | 100 |
| 27 | 100 | 100 |
| 38 | 100 | 100 |
| 40 | 100 | 100 |
| 47 | 100 | 100 |
| Comparative compound (a) | 0 | 0 |
| Comparative compound (b) | 100 | 20 |
| Comparative compound (d) | 0 | 0 |
| Non-treated | 0 | |

Table 5

| Test compound No. | Mortality (%) | |
|---|---|---|
| | 500 ppm | 50 ppm |
| 3 | 100 | 100 |
| 6 | 100 | 100 |
| 7 | 100 | 100 |
| 9 | 100 | 100 |
| 12 | 100 | 100 |
| 18 | 100 | 100 |
| 29 | 100 | 100 |
| 30 | 100 | 100 |
| 31 | 100 | 100 |
| 35 | 100 | 100 |
| 38 | 100 | 100 |
| 39 | 100 | 100 |
| 40 | 100 | 100 |
| 43 | 100 | 100 |
| 47 | 100 | 100 |
| Comparative compound (a) | 0 | 0 |
| Comparative compound (b) | 80 | 0 |
| Comparative compound (d) | 0 | 0 |
| Non-treated | 0 | |

Table 6

| Test compound No. | Mortality (%) | |
|---|---|---|
| | 500 ppm | 50 ppm |
| 7 | 100 | 100 |
| 29 | 100 | 100 |
| 30 | 100 | 100 |
| 35 | 100 | 100 |
| 38 | 100 | 100 |
| 40 | 100 | 100 |
| 44 | 100 | 100 |
| Comparative compound (a) | 100 | 100 |
| Comparative compound (c) | 100 | 100 |
| Non-treated | 0 | |

Comparative compound (a) is the following compound described in Japanese Laid-Open Patent

Publication No. 140577/1986.

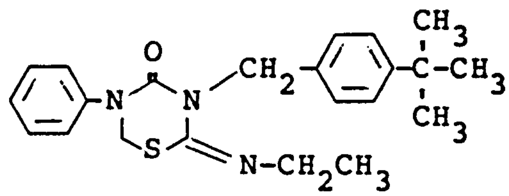

Comparative compound (b) is dichlorvos (DDVP) of the following formula.

$$(CH_3O)_2-\overset{O}{\overset{\|}{P}}-O-CH=CCl_2$$

Comparative compound (c) is diazinon of the following formula.

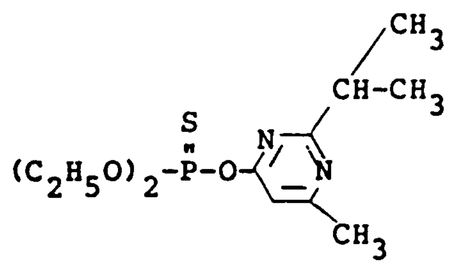

Comparative compound (d) is buprofezin of the following formula described in Japanese Laid-Open Patent Publication No. 3083/1979.

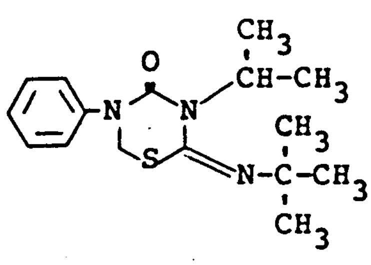

As is clearly seen from the foregoing description, the tetrahydro-1,3,5-thiadiazin-4-ones of general formula (I) or salts thereof provided by this invention show a very superior efficacy of controlling insect pests. Furthermore, agricultural chemicals containing these compounds have excellent characteristics as insecticides and are useful.

## Claims

### Claims for the following Contracting States : CH, DE, FR, GB, IT, LI, NL

**1.** A tetrahydro-1,3,5-thiadiazin-4-one represented by the following general formula (I)

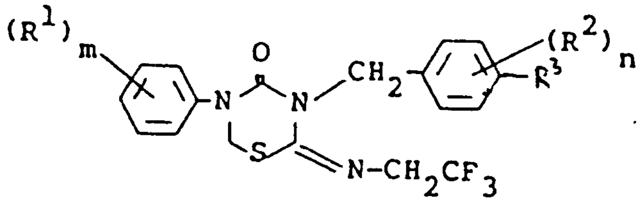

(I)

wherein each of $R^1$ and $R^2$ represents a halogen atom or an alkyl group having 1 to 4 carbon atoms, $R^3$ represents a halogen atom, or an alkyl group having 1 to 4 carbon atoms, a cycloalkyl group having 3 to 6 carbon atoms, an alkoxy having 1 to 6 carbon atoms, an acetyl, a phenoxy, a halo-substituted phenoxy, a benzyl, a benzyloxy, a phenylcarbonyl or a trifluoromethyl group, m represents 0, 1, 2 or 3, and n represents 0, 1 or 2;
and its salts.

2. A compound of claim 1 in which m in general formula (I) is 0.

3. A compound of claim 1 in which $R^1$ in general formula (I) is a halogen atom or an alkyl group with 1 to 4 carbon atoms.

4. A compound of claim 3 in which the halogen atom is a fluorine atom.

5. A compound of claim 4 in which the fluorine atom is substituted at the 2-position.

6. A compound of claim 3 in which the alkyl group is a methyl group.

7. A compound of claim 6 in which the methyl group is substituted at the 3-position.

8. A compound of claim 6 in which the methyl group is substituted at the 4-position.

9. A compound of any one of claims 1 to 8 in which $R^3$ in general formula (I) is a trifluoromethyl group.

10. A compound of any one of claims 1 to 8 in which $R^3$ in general formula (I) is a t-butyl group.

11. A process for producing a tetrahydro-1,3,5-thiadiazin-4-one represented by the following general formula (I)

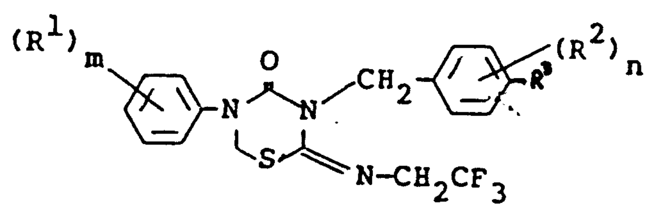

(I)

wherein $R^1$, $R^2$, $R^3$, m and n are as defined in claim 1,
or its salts,
which comprises reacting a compound represented by the following general formula (II)

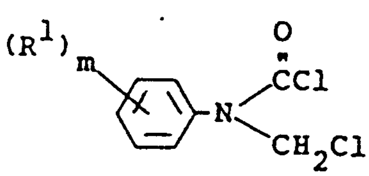

(II)

with a compound represented by the following general formula (III)

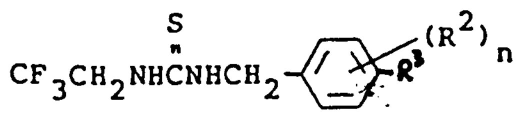

(III)

**12.** An insecticidal composition comprising (1) an effective amount of at least one tetrahydro-1,3,5-thiadiazin-4-one represented by the following general formula (I)

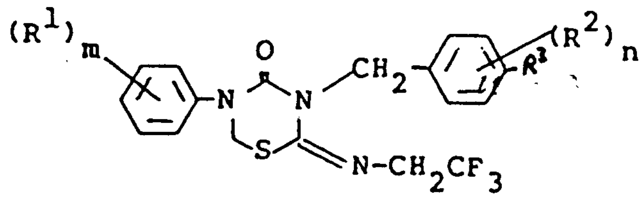

(I)

wherein $R^1$, $R^2$, $R^3$, m and n are as defined in claim 1,
or its salts, and (2) a carrier.

**13.** An insecticide of claim 12 in which m in general formula (I) is 0.

**14.** An insecticide of claim 12 in which $R^1$ in general formula (I) is a halogen atom or an alkyl group with 1 to 4 carbon atoms.

**15.** An insecticide of claim 14 in which the halogen atom is a fluorine atom.

**16.** An insecticide of claim 15 in which the fluorine atom is substituted at the 2-position.

**17.** An insecticide of claim 14 in which the alkyl group is a methyl group.

**18.** An insecticide of claim 17 in which the methyl group is substituted at the 3-position.

**19.** An insecticide of claim 17 in which the methyl group is substituted at the 4-position.

**20.** An insecticide of any one of claims 12 to 19 in which $R^3$ in general formula (I) is a trifluormethyl group.

**21.** An insecticide of any one of claims 12 to 19 in which $R^3$ in general formula (I) is a t-butyl group.

**Claims for the following Contracting State : ES**

**1.** A process for producing a tetrahydro-1,3,5-thiadiazin-4-one represented by the following general formula (I)

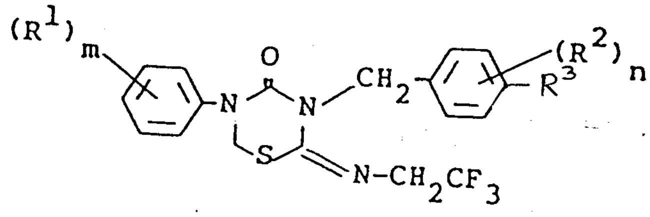

(I)

wherein each of $R^1$ and $R^2$ represents a halogen atom or an alkyl group having 1 to 4 carbon atoms, $R^3$ represents a halogen atom, or an alkyl group having 1 to 4 carbon atoms, a cycloalkyl group having 3 to 6 carbon atoms, an alkoxy having 1 to 6 carbon atoms, an acetyl, a phenoxy, a halo-substituted phenoxy, a benzyl, a benzyloxy, a phenylcarbonyl or a trifluoromethyl group, m represents 0, 1, 2 or 3, and n represents 0, 1 or 2;
or its salts,
which comprises reacting a compound represented by the following general formula (II)

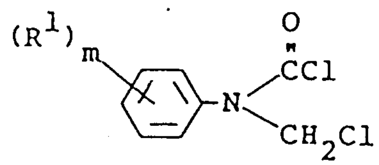

(II)

with a compound represented by the following general formula (III)

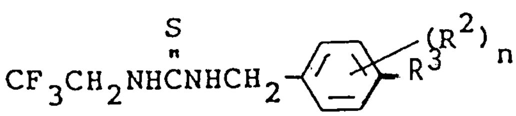

(III)

2. An insecticidal composition comprising (1) an effective amount of at least one tetrahydro-1,3,5-thiadiazin-4-one represented by the following general formula (I)

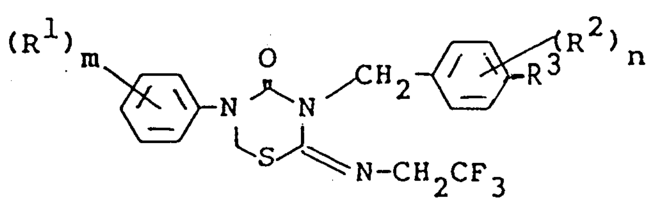

(I)

wherein $R^1$, $R^2$, $R^3$, m and n are as defined in claim 1,
or its salts, and (2) a carrier.

3. An insecticide of claim 2 in which m in general formula (I) is 0.

4. An insecticide of claim 2 in which $R^1$ in general formula (I) is a halogen atom or an alkyl group with 1 to 4 carbon atoms.

5. An insecticide of claim 4 in which the halogen atom is a fluorine atom.

6. An insecticide of claim 5 in which the fluorine atom is substituted at the 2-position.

7. An insecticide of claim 4 in which the alkyl group is a methyl group.

8. An insecticide of claim 7 in which the methyl group is substituted at the 3-position.

9. An insecticide of claim 7 in which the methyl group is substituted at the 4-position.

10. An insecticide of any one of claims 2 to 9 in which $R^3$ in general formula (I) is a trifluormethyl group.

**11.** An insecticide of any one of claims 2 to 9 in which $R^3$ in general formula (I) is a t-butyl group.

**Revendications**

**Revendications pour les Etats contractants suivants : CH, DE, FR, GB, IT, LI, NL**

**1.** Une tétrahydro-1,3,5-thiadiazine-4-one représentée par la formule générale (I) suivante

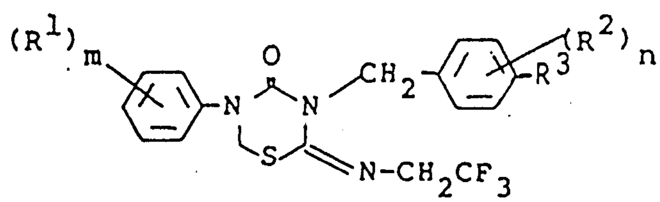

(I)

dans laquelle chacun de $R^1$ et $R^2$ représente un atome d'halogène ou un groupe alcoyle ayant 1 à 4 atomes de carbone, $R^3$ représente un atome d'halogène ou un groupe alcoyle ayant 1 à 4 atomes de carbone, un groupe cycloalcoyle ayant 3 à 6 atomes de carbone, un alcoxy ayant 1 à 6 atomes de carbone, un acétyle, un phénoxy, un phénoxy halogéno-substitué, un benzyle, un benzyloxy, un phénylcarbonyle ou un groupe trifluorométhyle, m représente 0, 1, 2 ou 3 et n représente 0, 1 ou 2 ; et ses sels.

**2.** Un composé selon la revendication 1, dans lequel m dans la formule générale (I) est 0.

**3.** Un composé selon la revendication 1, dans lequel $R^1$ dans la formule générale (I) est un atome d'halogène ou un groupe alcoyle ayant 1 à 4 atomes de carbone.

**4.** Un composé selon la revendication 3, dans lequel l'atome d'halogène est un atome de fluor.

**5.** Un composé selon la revendication 4, dans lequel l'atome de fluor substitue la position 2.

**6.** Un composé selon la revendication 3, dans lequel le groupe alcoyle est un groupe méthyle.

**7.** Un composé selon la revendication 6, dans lequel le groupe méthyle substitue la position 3.

**8.** Un composé selon la revendication 6, dans lequel le groupe méthyle substitue la position 4.

**9.** Un composé selon l'une quelconque des revendications 1 à 8, dans lequel $R^3$ dans la formule générale (I) est un groupe trifluorométhyle.

**10.** Un composé selon l'une quelconque des revendications 1 à 8, dans lequel $R^3$ dans la formule générale (I) est un groupe tert-butyle.

**11.** Un procédé pour produire une tétrahydro-1,3,5-thiadiazine-4-one représentée par la formule générale (I) suivante

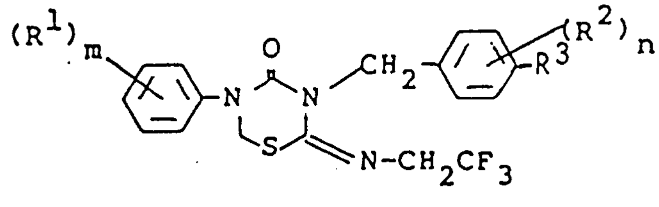

(I)

dans laquelle $R^1$, $R^2$, $R^3$, m et n sont comme définis dans la revendication 1, ou ses sels, qui comprend la réaction d'un composé représenté par la formule générale (II) suivante

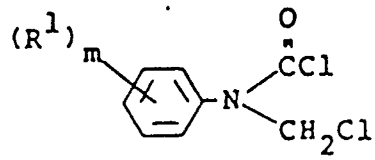

(II)

avec un composé représenté par la formule générale (III) suivante

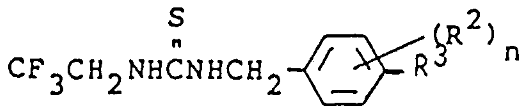

(III)

**12.** Une composition insecticide comprenant (1) une quantité efficace d'au moins une tétrahydro-1,3,5-thiadiazine-4-one représentée par la formule générale (I) suivante

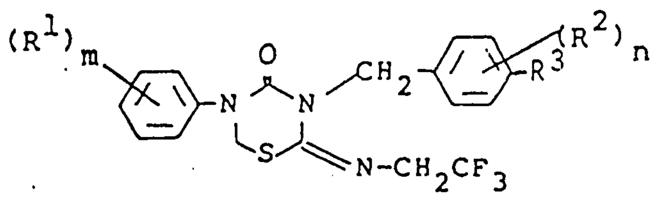

(I)

dans laquelle $R^1$, $R^2$, $R^3$, m et n sont comme définis dans la revendication 1, ou ses sels, et (2) un support.

**13.** Un insecticide selon la revendication 12, dans lequel m dans la formule générale (I) est 0.

**14.** Un insecticide selon la revendication 12, dans lequel $R^1$ dans la formule générale (I) est un atome d'halogène ou un groupe alcoyle ayant 1 à 4 atomes de carbone.

**15.** Un insecticide selon la revendication 14, dans lequel l'atome d'halogène est un atome de fluor.

**16.** Un insecticide selon la revendication 15, dans lequel l'atome de fluor substitue la position 2.

**17.** Un insecticide selon la revendication 14, dans lequel le groupe alcoyle est un groupe méthyle.

**18.** Un insecticide selon la revendication 17, dans lequel le groupe méthyle substitue la position 3.

**19.** Un insecticide selon la revendication 17, dans lequel le groupe méthyle substitue la position 4.

**20.** Un insecticide selon l'une quelconque des revendications 12 à 19, dans lequel $R^3$ dans la formule générale (I) est un groupe trifluorométhyle.

**21.** Un insecticide selon l'une quelconque des revendications 12 à 19, dans lequel $R^3$ dans la formule générale (I) est un groupe tert-butyle.

**Revendications pour l'Etat contractant suivant : ES**

**1.** Un procédé pour préparer une tétrahydro-1,3,5-thiadiazine-4-one représentée par la formule générale (I) suivante

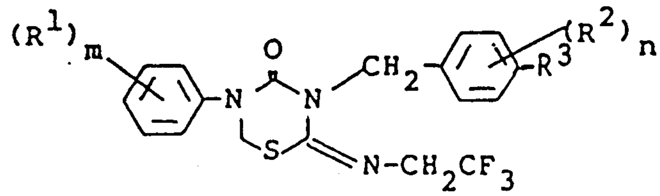

(I)

dans laquelle chacun de $R^1$ et $R^2$ représente un atome d'halogène ou un groupe alcoyle ayant 1 à 4 atomes de carbone, $R^3$ représente un atome d'halogène ou un groupe alcoyle ayant 1 à 4 atomes de carbone, un groupe cycloalcoyle ayant 3 à 6 atomes de carbone, un alcoxy ayant 1 à 6 atomes de carbone, un acétyle, un phénoxy, un phénoxy halogéno-substitué, un benzyle, un benzyloxy, un phénylcarbonyle ou un groupe trifluorométhyle, m représente 0, 1, 2 ou 3 et n représente 0, 1 ou 2 ; ou ses sels,
qui comprend la réaction d'un composé représenté par la formule générale (II) suivante

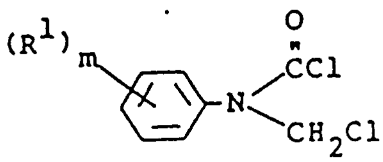

(II)

avec un composé représenté par la formule générale (III) suivante

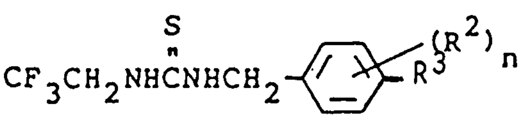

(III)

**2.** Une composition insecticide comprenant (1) une quantité efficace d'au moins une tétrahydro-1,3,5-thiadiazine-4-one représentée par la formule générale (I) suivante

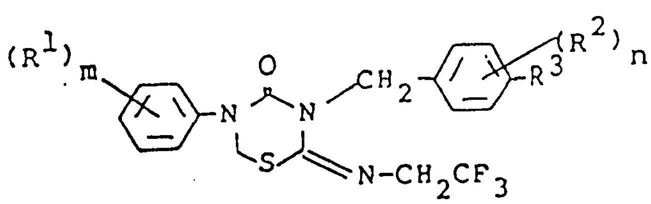

(I)

dans laquelle $R^1$, $R^2$, $R^3$, m et n sont comme définis dans la revendication 1, ou ses sels, et (2) un support.

**3.** Un insecticide selon la revendication 2, dans lequel m dans la formule générale (I) est 0.

**4.** Un insecticide selon la revendication 2, dans lequel $R^1$ dans la formule générale (I) est un atome d'halogène ou un groupe alcoyle ayant 1 à 4 atomes de carbone.

**5.** Un insecticide selon la revendication 4, dans lequel l'atome d'halogène est un atome de fluor.

**6.** Un insecticide selon la revendication 5, dans lequel l'atome de fluor substitue la position 2.

**7.** Un insecticide selon la revendication 4, dans lequel le groupe alcoyle est un groupe méthyle.

**8.** Un insecticide selon la revendication 7, dans lequel le groupe méthyle substitue la position 3.

**9.** Un insecticide selon la revendication 7, dans lequel le groupe méthyle substitue la position 4.

**10.** Un insecticide selon l'une quelconque des revendications 2 à 9, dans lequel $R^3$ dans la formule générale (I) est un groupe trifluorométhyle.

**11.** Un insecticide selon l'une quelconque des revendications 2 à 9, dans lequel $R^3$ dans la formule générale (I) est un groupe tert-butyle.

**Patentansprüche**

**Patentansprüche für folgende Vertragsstaaten : CH, DE, FR, GB, IT, LI, NL**

**1.** Tetrahydro-1,3,5-thiadiazin-4-on der folgenden, allgemeinen Formel (I)

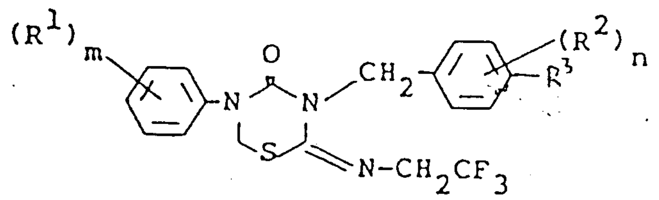

(I)

worin ein jedes von $R^1$ und $R^2$ ein Halogenatom oder eine Alkylgruppe mit 1 bis 4 Kohlenstoffatomen bedeutet, $R^3$ für ein Halogenatom oder eine Alkylgruppe mit 1 bis 4 Kohlenstoffatomen, eine Cycloalkylgruppe mit 3 bis 6 Kohlenstoffatomen, eine Alkoxygruppe mit 1 bis 6 Kohlenstoffatomen, eine Acetyl-, eine Phenoxy-, eine Halo-substituierte Phenoxy-, eine Benzyl-, eine Benzyloxy-, eine Phenylcarbonyl- oder eine Trifluormethylgruppe steht, m 0, 1, 2 oder 3 bedeutet und n für 0, 1 oder 2 steht, und dessen Salze.

**2.** Verbindung gemäß Anspruch 1, worin m in der allgemeinen Formel (I) für 0 steht.

**3.** Verbindung gemäß Anspruch 1, worin $R^1$ in der allgemeinen Formel (I) für ein Halogenatom oder eine Alkylgruppe mit 1 bis 4 Kohlenstoffatomen steht.

**4.** Verbindung gemäß Anspruch 3, worin das Halogenatom ein Fluoratom ist.

**5.** Verbindung gemäß Anspruch 4, worin das Fluoratom als Substituent in der 2-Stellung vorliegt.

**6.** Verbindung gemäß Anspruch 3, worin die Alkylgruppe eine Methylgruppe ist.

**7.** Verbindung gemäß Anspruch 6, worin die Methylgruppe als Substituent in der 3-Stellung vorliegt.

**8.** Verbindung gemäß Anspruch 6, worin die Methylgruppe als Substituent in der 4-Stellung vorliegt.

**9.** Verbindung gemäß einem der Ansprüche 1 bis 8, worin $R^3$ in der allgemeinen Formel (I) für eine Trifluormethylgruppe steht.

**10.** Verbindung gemäß einem der Ansprüche 1 bis 8, worin $R^3$ in der allgemeinen Formel (I) eine tert.-Butylgruppe ist.

**11.** Verfahren zur Herstellung eines Tetrahydro-1,3,5-thiadiazin-4-ons der folgenden, allgemeinen Formel (I)

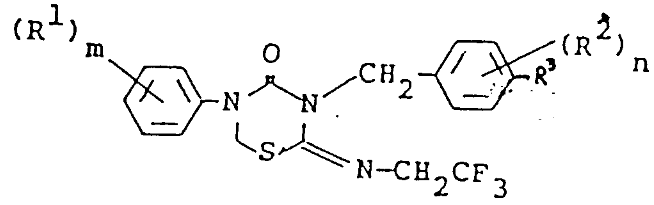

(I)

worin $R^1$, $R^2$, $R^3$, m und n wie in Anspruch 1 definiert sind, oder eines seiner Salze, das die Reaktion einer Verbindung der folgenden, allgemeinen Formel (II)

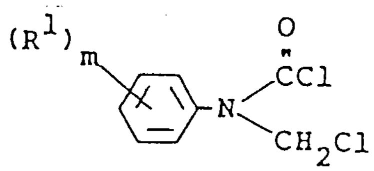

(II)

mit einer Verbindung der folgenden, allgemeinen Formel (III)

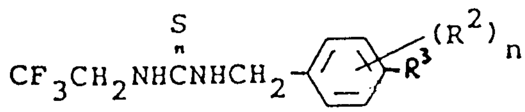

(III)

umfaßt.

12. Insektizide Zusammensetzung, umfassend (1) eine wirksame Menge zumindest eines Tetrahydro-1,3,5-thiadiazin-4-ons der folgenden, allgemeinen Formel (I)

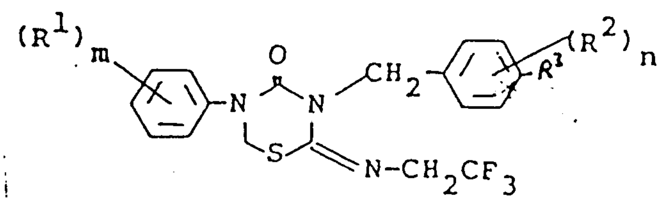

(I)

worin $R^1$, $R^2$, $R^3$, m und n wie in Anspruch 1 definiert sind, oder seine Salze und (2) einen Träger.

13. Insektizid gemäß Anspruch 12, worin m in der allgemeinen Formel (I) für 0 steht.

14. Insektizid gemäß Anspruch 12, worin $R^1$ in der allgemeinen Formel (I) ein Halogenatom oder eine Alkylgruppe mit 1 bis 4 Kohlenstoffatomen bedeutet.

15. Insektizid gemaß Anspruch 14, worin das Halogenatom ein Fluoratom ist.

16. Insektizid gemäß Anspruch 15, worin das Fluoratom als Substituent in der 2-Stellung vorliegt.

17. Insektizid gemäß Anspruch 14, worin die Alkylgruppe eine Methylgruppe ist.

18. Insektizid gemäß Anspruch 17, worin die Methylgruppe als Substituent in der 3-Stellung vorliegt.

**19.** Insektizid gemäß Anspruch 17, worin die Methylgruppe als Substituent in der 4-Stellung vorliegt.

**20.** Insektizid gemäß einem der Ansprüche 12 bis 19, worin $R^3$ in der allgemeinen Formel (I) eine Trifluormethylgruppe bedeutet.

**21.** Insektizid gemäß einem der Ansprüche 12 bis 19, worin $R^3$ in der allgemeinen Formel (I) eine tert.-Butylgruppe wiedergibt.

**Patentansprüche für folgenden Vertragsstaat : ES**

**1.** Verfahren zur Herstellung eines Tetrahydro-1,3,5-thiadiazin-4-ons der folgenden, allgemeinen Formel (I)

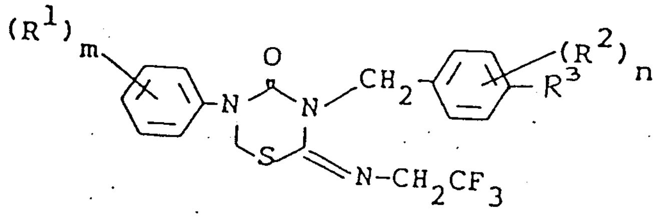

(I)

worin ein jedes von $R^1$ und $R^2$ ein Halogenatom oder eine Alkylgruppe mit 1 bis 4 Kohlenstoffatomen bedeutet, $R^3$ für ein Halogenatom oder eine Alkylgruppe mit 1 bis 4 Kohlenstoffatomen, eine Cycloalkylgruppe mit 3 bis 6 Kohlenstoffatomen, eine Alkoxygruppe mit 1 bis 6 Kohlenstoffatomen, eine Acetyl-, eine Phenoxy-, eine Halo-substituierte Phenoxy-, eine Benzyl-, eine Benzyloxy-, eine Phenylcarbonyl- oder eine Trifluormethylgruppe steht, m 0, 1, 2 oder 3 bedeutet und n für 0, 1 oder 2 steht, oder von dessen Salzen, das die Umsetzung einer Verbindung der folgenden, allgemeinen Formel (II)

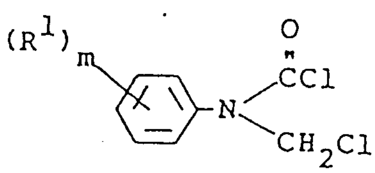

(II)

mit einer Verbindung der folgenden, allgemeinen Formel (III)

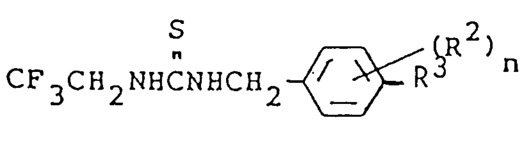

(III)

umfaßt.

**2.** Insektizide Zusammensetzung, umfassend (1) eine wirksame Menge zumindest eines Tetrahydro-1,3,5-thiadiazin-4-ons der folgenden, allgemeinen Formel (I)

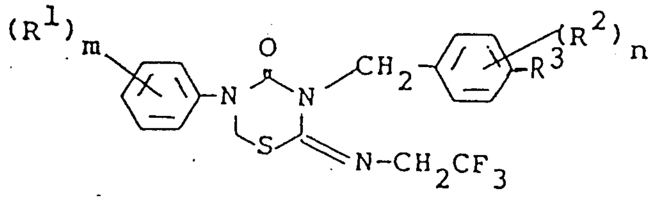

(I)

worin $R^1$, $R^2$, $R^3$, m und n wie in Anspruch 1 definiert sind, oder seiner Salze und (2) einen Träger.

**3.** Insektizid gemäß Anspruch 2, worin m in der allgemeinen Formel (I) für 0 steht.

**4.** Insektizid gemäß Anspruch 2, worin $R^1$ in der allgemeinen Formel (I) für ein Halogenatom oder eine Alkylgruppe mit 1 bis 4 Kohlenstoffatomen steht.

**5.** Insektizid gemäß Anspruch 4, worin das Halogenatom ein Fluoratom ist.

**6.** Insektizid gemäß Anspruch 5, worin das Fluoratom als Substituent in der 2-Stellung vorliegt.

**7.** Insektizid gemäß Anspruch 4, worin die Alkylgruppe eine Methylgruppe ist.

**8.** Insektizid gemäß Anspruch 7, worin die Methylgruppe als Substituent in der 3-Stellung vorliegt.

**9.** Insektizid gemäß Anspruch 7, worin die Methylgruppe als Substituent in der 4-Stellung vorliegt.

**10.** Insektizid gemäß einem der Ansprüche 2 bis 9, worin $R^3$ in der allgemeinen Formel (I) für eine Trifluormethylgruppe steht.

**11.** Insektizid gemäß einem der Ansprüche 2 bis 9, worin $R^3$ in der allgemeinen Formel (I) für eine tert.-Butylgruppe steht.